# EUROPÄISCHE PATENTSCHRIFT

(11) **EP 0 857 709 B1**
(45) Veröffentlichungstag und Bekanntmachung des Hinweises auf die Patenterteilung: **16.08.2001**
(21) Anmeldenummer: 97121887.0
(22) Anmeldetag: 12.12.1997
(51) Int. Cl.: C07C 29/00, C08F 8/00, C07C 45/00, B01J 31/00

(54) **Verfahren zur Herstellung von 1,3-Propandiol**
Process for the preparation of 1,3-propanediol
Procédé de préparation de 1,3-propanediol

(30) Priorität: 30.01.1997 DE 19703383
(43) Veröffentlichungstag der Anmeldung: 12.08.1998
(73) Patentinhaber: E.I. DU PONT DE NEMOURS AND COMPANY, Wilmington Delaware 19898 (US)
(72) Erfinder: Brossmer, Christoph, Dr., 60318 Frankfurt (DE); Arntz, Dietrich, Dr., 36695 Mobile, Alabama (US)
(74) Vertreter: Münsterer, Heribert

(56) Entgegenhaltungen:
- EP-A- 0 412 337
- EP-A- 0 464 458
- EP-A- 0 487 903
- EP-A- 0 713 853
- US-A- 3 536 763
- DATABASE WPI Week 9632 Derwent Publications Ltd., London, GB; AN 96-318886 XP002061840 "Preparation of hydroxyalkanal - by hydration of unsatd. aldehyde in presence of carboxylic acid" & JP 08 143 502 A (NIPPON SHOKUBAI) , 4.Juni 1996
- CHEMICAL ABSTRACTS, vol. 96, no. 23, 7.Juni 1982 Columbus, Ohio, US; abstract no. 199037c, A. MUREL : "Synthesis and properties of ampholyte-supports" Seite 612; XP002063242 & MATER. VSES. SHK. ELEKTROFOR. METODAM. ANAL., 1980, Seiten 106-113,
- CHEMICAL ABSTRACTS, vol. 123, no. 26, 25.Dezember 1995 Columbus, Ohio, US; abstract no. 352823d, T. SIYAM ET AL: "Synthesis and characterization of polyacrylamide-acrylic acid resin and its ude for treatment of radiotive liquid waste" XP002061949 & MACROMOL. REP. , Bd. a32, 1995, Seiten 871-879,
- CHEMICAL ABSTRACTS, vol. 125, no. 22, 25.November 1996 Columbus, Ohio, US; abstract no. 279588r, H. MATSUYAMA ET AL: "Facilitated transport of carbon dioxide through functional membranes prepared by plasma greaft polymerization using amines as carrier" Seite 235; XP002063243 & ACS SYMP. SER., Nr. 642, 1996, Seiten 252-269,

## Beschreibung

Die Erfindung betrifft ein Verfahren zur Herstellung von 1,3- Propandiol durch Hydratisierung von Acrolein in Gegenwart eines Ionenaustauscherharzes mit nachfolgender katalytischer Hydrierung des 3-Hydroxypropionaldehyds.

1,3-Propandiol besitzt als Monomerbaustein für Polyester und Polyurethane sowie als Ausgangsstoff für die Synthese cyclischer Verbindungen vielseitige Anwendungsmöglichkeiten.

Es ist bekannt, 1,3-Propandiol herzustellen, indem man Acrolein zu 3-Hydroxypropionaldehyd hydratisiert und anschließend zu 1,3-Propandiol hydriert.

Gemäß US-PS 2,434,110 kann man Acrolein in Gegenwart eines sauren Katalysators hydratisieren, wobei 3-Hydroxypropionaldehyd gebildet wird. Die Umsetzung erfolgt bei erhöhter Temperatur unter Verwendung einer 5 bis 30 gew.-%igen Lösung von Acrolein in Wasser und einer Säure oder sauren Salzen als Katalysator. Das bei der Hydratisierung erhaltene Reaktionsgemisch wird, gegenenfalls nach Entfernung von nicht umgesetztem Acrolein, in Gegenwart bekannter Hydrierkatalysatoren hydriert. Für die Hydrierung des 3-Hydroxypropionaldehyds zu 1,3- Propandiol sind Katalysatoren, welche ein oder mehrere hydrierwirksame Metalle, wie z.B. Fe, Co, Ni, Cu, Ag, Mo, W, V, Cr, Rh, Pd, Os, Ir oder Pt enthalten, geeignet.

Nachteilig an dem bekannten Verfahren gemäß US-PS 2,434,110 sind die niedrigen Ausbeuten an 1,3- Propandiol, welche insbesondere auf Acrolein verbrauchende Nebenreaktionen während der Hydratisierungsstufe zurückzuführen sind. Die Selektivität der mineralsauer katalysierten Hydratisierung ist zudem stark von dem Acrolein-Umsatz abhängig. Um eine akzeptable Selektivität zu erreichen, beendet man die Hydratisierung bei niedrigem Acrolein-Umsatz, wobei jedoch nur eine geringe Raum-Zeit-Ausbeute erzielt werden kann.

Es sind Versuche bekannt, die Nachteile des Verfahrens zu mindern. So wird versucht durch Anlagern von niederen Carbonsäuren an Acrolein unter Erhitzen (US-PS 2,638,479) oder in Gegenwart eines basischen Ionenaustauscherharzes Carbonsäure-ester des 3-Hydroxypropionaldehyds, die zu den entsprechenden Estern des 1,3- Propandiols hydriert werden können, zu erhalten. Nachteilig sind bei diesen Verfahren die zusätzlich notwendigen Schritte zur Verseifung des Esters und zur Recyclierung der Carbonsäure sowie die unerwünschte Bildung von n-Propanol und seinen Carbonsäureestern bei der Hydrierung (DE-OS 20 57 399). Bekannt ist auch die Hydratisierung von Acrolein mit Kohlendioxid als Katalysator; ein Verfahren,welches jedoch lange Reaktionszeiten erfordert (DE-OS 19 05 823).

Es wurde festgestellt, daß unter Verwendung von z.B. Phosphorsäure oder Dihydrogenphosphaten als Katalysator zwar die Hydratisierung von Acrolein durchgeführt werden kann, bei der anschließenden Hydrierung aber Probleme auftreten, wenn die so erhaltene Hydroxypropionaldehyd-Lösung ohne sorgfältige Abtrennung des Hydratisierungskatalysators hydriert wird (EP-A 0 487 903).

Bei Verwendung von an sich sehr wirksamen Nickel-Hydrierkatalysatoren wird bei wiederholtem Einsatz des Katalysators dieser schneller desaktiviert, als wenn ein säure- und salzfreies Reaktionsgemisch hydriert wird. Dies führt zu einem erhöhten Katalysatorverbrauch. Zusätzlich kommt es durch die Anwesenheit des Hydratisierungskatalysators während der destillativen Aufarbeitung zu Produktverlusten durch Zersetzung bzw. im Falle einer vorangehenden Neutralisation zu Verstopfungen und Verkrustungen in der Anlage. Auch ist die Beseitigung von Destillationsrückständen bei einem Gehalt an anorganischen Salzen schwieriger und damit teurer als ohne diese Salze (EP-A 0 487 903).

Die Nachteile lassen sich zum Teil vermeiden, wenn man den Hydratisierungskatalysator mittels Ionenaustauschern vor der Hydrierung aus dem Reaktionsgemisch entfernt oder 3-Hydroxypropionaldehyd extraktiv aus dem Reaktionsgemisch abtrennt und dann hydriert. Beide alternativen Maßnahmen zur Reduzierung des Verbrauchs an teurem Hydrierkatalysator machen aber zusätzliche Einrichtungen erforderlich. Sie führen zu einem höheren Energieverbrauch und zu Abwasserproblemen und erhöhen somit die Herstellkosten für 1,3-Propandiol (EP-A 0 487 903).

Gemäß der US-PS 3,536,763 wird die Hydratisierung von Acrolein in Gegenwart schwachsaurer Kationenaustauscherharze, deren funktionelle Gruppen nur Carboxylgruppen sind, bei 40 bis 120 °C durchgeführt. Die Ausbeuten an 3-Hydroxypropionaldehyd werden mit etwa 80 % angegeben, wobei die Ausbeuten vom Acrolein-Umsatz im Bereich von 25 bis 65 % praktisch nicht abhängig sein sollen. Dieses Dokument umfaßt auch die an sich bekannte Hydrierung des 3-Hydroxypropionaldehyds zu 1,3- Propandiol.

Bei der Nacharbeitung des Verfahrens der US-PS 3,536,763 konnte zwar die katalytische Wirksamkeit der Ionenaustauscherharze mit Carboxylgruppen bestätigt werden, der Grad der Wirksamkeit ließ aber die Verwendung dieser Ionenaustauscher in technischen Anlagen als nicht geeignet erscheinen. Es zeigt sich nämlich, daß diese Katalysatoren höhere Temperaturen und längere Reaktionszeiten erfordern, was der erwünschten hohen Raum-Zeit-Ausbeute und hohen Selektivität zuwiderläuft.

Es ist weiterhin bekannt, die Hydratisierung mit der Herstellung von 1,3- Propandiol in Gegenwart eines chelatbildenden Ionenaustauschers durchzuführen (EP-A 0 487 903).

Die Verwendung der chelatbildenden Ionenaustauscher hat den Nachteil, daß diese Harze bei der Überführung von der handelsüblichen Na-Form in die katalytisch aktive H-Form ("Aktivierung") unerwünschten Volumenveränderungen /Volumenkontraktion) von bis zu 45 % unterworfen sind, welche im technischen Einsatz Probleme bereiten und zu langen Katalysatorwechselzeiten führen. Nachteilig ist auch die teilweise niedrige Beladungsdichte mit katalytisch aktiven, chelatbildenden Ankergruppen (z.B. vom Iminodiessigsäuretyp), welche sich in einer niedrigen (limitierten) Totalkapazität (meq. H+/ml) der Harze äußert. Letztere ist eine für die katalytische Aktivität der Harze wichtige Einflußgröße.

Es ist weiterhin bekannt, im Zusammenhang mit der Herstellung von 1,3- Propandiol, die Hydratisierung des Acroleins zur Herstellung von 3-Hydroxypropionaldehyd in Gegenwart von Kationenaustauscherharzen, die Phosphonsäuregruppen aufweisen, durchzuführen (EP-A 0 412 337).

Der Einsatz der Phosphonsäuregruppen enthaltenden Kationenaustauscherharzen hat den Nachteil, daß diese Harze bei der genauen Nacharbeitung im Langzeittest gemäß der EP-A 0 412 337 eine niedrigere Selektivität aufweisen und zu mehr Nebenprodukten führen, als die in der EP-A 0 487 903 aufgeführten chelatbildenden Ionenaustauscher. Darüberhinaus ist die Aktivität dieser Harze ebenfalls mit einer unerwünschten Volumenkontraktion verknüpft, welche technische Probleme und längere Katalysatorwechselzeiten mit sich bringt.

A. Murel, "Synthesis and properties of ampholyte-supports", Mater. Vses. Shk. Elektrofor. Metodam Anal., 1980, S. 106-113 (Chem. Abstr. Bd. 96, Nr. 23, 199037c) beschreibt die Behandlung von H₂N(CH₂CH₂NH)ₙH (n = 3-5) mit CH₂(NHCOCHCH₂)₂ im Verhältnis 2 : 1 und anschließend mit Acrylsäure, was in für die Proteinelektrophorese geeigneten Ionenaustauschern resultiert.

EP-A-713 853 beschreibt u.a. ein Verfahren zur Herstellung von Hydroxyalkanalen durch Hydratisierung von ungesättigten Aldehyden (z.B. Acrolein) in Gegenwart eines Harzes auf Carbonsäure-Basis, z.B. eines Copolymerisats aus Acrylsäure und Acrylamid.

Aufgabe der vorliegenden Erfindung ist, ein verbessertes Verfahren zur Herstellung von 1,3- Propandiol durch Hydratisierung von Acrolein in Gegenwart von Ionenaustauscherharzen mit nachfolgenden katalytischer Hydrierung zur Verfügung zu stellen, das in der Hydratisierungsstufe mit guter Selektivität und möglichst hoher Raum-Zeit-Ausbeute durchgeführt werden kann.

Eine weitere Aufgabe ist es, im Rahmen der AcroleinHydratisierung Ionenaustauscher-Katalysatoren zur Verfügung zu stellen, die die vorgenannten Nachteile der oben aufgeführten Harze nicht aufweisen und eine hohe Totalkapazität besitzen.

Das bei der Hydratisierung erhaltene Reaktionsgemisch sollte auch den Hydrierkatalysator möglichst wenig desaktivieren, um die Wiederverwendung des Hydrierkatalysators in Folgechargen zu ermöglichen, beziehungsweise die Standzeit eines Festbett-Hydrierkatalysators zu verlängern und damit die Wirtschaftlichkeit des Verfahrens zu verbessern.

Gegenstand der Erfindung ist ein Verfahren nach Anspruch 2 zur Herstellung von 1,3- Propandiol durch Hydratisierung von Acrolein in Gegenwart eines erfindungsgemäßen, als Hydratisierungskatalysator verwendeten Ionenaustauschers nach Anspruch 1 unter Bildung von 3-Eydroxypropionaldehyd, wobei Acrolein und Wasser im Gewichtsverhältnis von 1:2 bis 1:20 bei 30 bis 120 °C und einem Druck im Bereich von 1 bis 20 bar umgesetzt werden, Abtrennung des Ionenaustauschers und, soweit vorhanden, des nicht umgesetzten Acroleins aus dem Reaktionsgemisch und anschließende katalytische Hydrierung des 3-Hydroxypropionaldehyds unter an sich bekannten Bedingungen in flüssiger oder gasförmiger Phase unter Verwendung üblicher Hydrierkatalysatoren, Weiterer Gegenstand der Erfindung ist ein Ionenaustauscher nach Anspruch 1, umfassend ein Polyamin-/Polycarbonsäureharz, dadurch gekennzeichnet, daß ein von einer quervernetzten Polyacrylamid-Matrix abgeleitetes Polyaminharz mit Acrylsäure, Acrylsäurederivaten oder Salzen einer ω-Halogenalkansäure umgesetzt ist, wobei die Polyacrylamid-Matrix durch Reaktion einer Polyacrylatvorstufe mit Tetraethylenpentamin oder Triethylentetramin erhältlich ist.

Die Fa. BAYER bietet für die selektive Sulfat-Entfernung den Anionenaustauscher Lewatit® E304/88 an. Dabei handelt es sich um ein Polyaminharz auf einer vernetzten Polyacrylamid-Matrix. Nach Auskunft von BAYER wird es durch Umsetzung einer Polyacrylat-Vorstufe (z.B. Polyacrylsäureester) mit Tetraethylenpentamin oder Triethylentetramin hergestellt. Hierbei bildet eine der Aminogruppen zur Verankerung an die Polyacrylat-Matrix eine Amidbindung aus. Die restlichen Aminogruppen sind frei und prägen die Polyaminfunktionalität des Harzes.

Das Polyaminharz ist für die Acrolein-Hydratisierung zunächst ungeeignet, da es als Anionenaustauscher in der OH- bzw. Cl-Form vorliegt und unter diesen Bedingungen nur die unerwünschte Acrolein-Polymerisation katalysiert. Durch Reaktionen mit Acrylsäure, Acrylsäurederivaten oder dem Salz einer ω-Halogencarbonsäure (Natrium-2-chloracetat, Na-3-brompropionat) erhält man jedoch mit Carbonsäuregruppen belegte Harze, die hohe H⁺-Kapazitäten aufweisen. Die Synthese der erfindungsgemäßen Polyaminpolycarbonsäureharze erfolgt schematisch nach folgenden Gleichungen, wobei aus Gründen der Übersichtlichkeit nur die Reaktion an einer Aminogruppe abgebildet ist:

### Umsetzung mit Acrylsäure

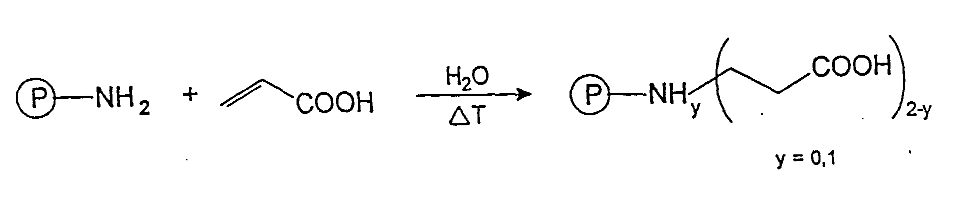

### Umsetzung mit einem ω-Halogencarbonsäure-Salz

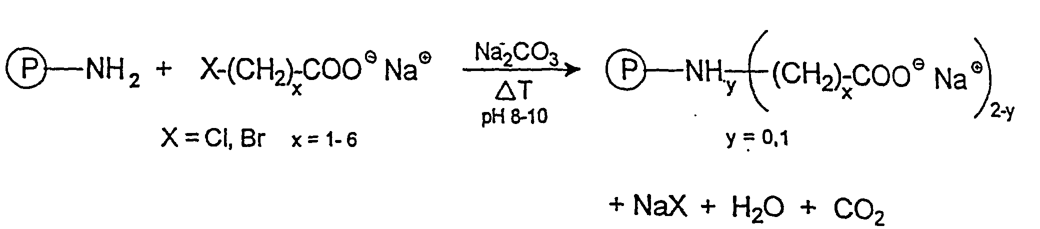

Im erstem Fall werden Aminogruppen an die Doppelbindung der Acrylsäure bzw. deren Derivate addiert, wobei Amino- bzw. Iminodipropionsäure-Gruppen entstehen. Im zweiten Fall können durch Kondensation (HX-Abspaltung) Amino- bzw. Iminodicarbonsäuren unterschiedlicher Kettenlänge aufgebaut werden. Charakteristisch für diese Derivatisierungen ist die Umpolung des Harzes, d.h. es wird der Anionenaustauscher in einen Kationenaustauscher umgewandelt.

Die erfindungsgemäß für die Harz-Derivatisierung verwendete Temperatur liegt bei der Umsetzung mit Acrylsäure bei 0 - 150 °C, vorzugsweise 20 - 130 °C. Der Einsatz von Acrylsäure/Methacrylsäure erfolgt in stabilisierter Form in Abwesenheit oder in Gegenwart von Wasser. Bei der Umsetzung in einem Säure/Wassergemisch kann der Acrylsäure-/MethacrylsäureAnteil zwischen 5 - 100 Gew.-%, vorzugsweise 30 - 90 Gew.-% betragen. Als Acrylsäurederivate können beispielsweise Acrylsäureester, Acrylsäureanhydride, Acrylsäurechloride und/oder Acrylsäureamide eingesetzt werden. Durch eine anschliessende Verseifung kann die Carboxylfunktion freigesetzt werden 5 (Entschützen).

Die Umsetzung mit Salzen der ω-Halogencarbonsäure erfolgt bei 0 - 60 °C, vorzugsweise 10- 60 °C, und ist in der Literatur R. Hering "Chelatbildende Ionenaustauscher" (Akademie-Verlag, Berlin 1967) für die Synthese sog. "Chelationenaustauscher" beschrieben. Gängige ω-Halogencarbonsäure-Salze sind Natrium-2-chloracetat und Natrium-3-chlorpropionat sowie höhere Homologe.

Die erfindungsgemäßen Polyaminpolycarbonsäure-Harze zeigen überraschenderweise nur sehr geringe Volumenänderungen bei der Überführung von der Na- in die katalytisch aktive H-Form und vice versa.

Zur Hydratisierung des Acroleins werden Acrolein und Wasser in einem Gewichtsverhältnis von 1:2 bis 1:20, insbesondere 1:3 bis 1:10 und bevorzugt 1:3 bis 1:6, der Hydratisierungsstufe zugeführt. Die Umsetzung zum 3-Hydroxypropionaldehyd erfolgt im Temperaturbereich von 30 bis 120 °C. Eine Temperatur im Bereich von 40 bis 90 °C wird bevorzugt. Besonders bevorzugt erfolgt die Hydratisierung bei 50 bis 80 °C. Eine Temperatur unter 40 °C führt im allgemeinen zu langen Reaktionszeiten. Eine Temperatur oberhalb 90 °C führt zu einer verminderten Selektivität und Problemen bezüglich der Standzeit der Austauscherharze.

In dem Temperaturbereich unterhalb des Siedepunkts des Acroleins kann die Umsetzung bei Normaldruck oder mäßigem Druck erfolgen. Bei Umsetzungstemperaturen um oder oberhalb des Siedepunkts von Acrolein wird man unter einem Druck im Bereich von etwa 2 bis 20 bar arbeiten. Im bevorzugten Temperaturbereich von 40 bis 90 °C hat sich ein Druck im Bereich von 2 bis 5 bar als geeignet erwiesen.

Die Hydratisierung wird im allgemeinen bis zu einem Acrolein-umsatz im Bereich von 30 bis 90 % oder darüber durchgeführt. Ein Umsatz von 40 bis 90 % und insbesondere 50 bis 80 % wird. bevorzugt.

Zweckmäßigerweise können dem Acrolein-Wasser-Gemisch Polymerisationsinhibitoren, wie z.B. Hydrochinon, Hydrochinonmonome-thylether oder butylierte Phenole, in einer Menge von 100 bis 2000 ppm zugegeben werden.

Die Hydratisierung kann diskontinuierlich oder kontinuierlich in an sich bekannten Reaktoren, wie z.B. Rührreaktoren, Schlaufenreaktoren, Schwebebett-, Wirbelbett- oder Festbettreaktoren durchgeführt werden. Die zuletzt genannten Reaktoren werden gegenüber den Schlaufen- und Rührreaktoren bevorzugt. Die Durchflußgeschwindigkeit durch einen Festbettreaktor, der den Ionenaustauscher enthält und mit einem heizbaren Mantel versehen ist, sowie die Reaktionstemperatur wird man vorzugsweise so aufeinander abstimmen, daß durch einmalige Passage des Reaktionsgemisches durch den Reaktor der gewünschte Acrolein-Umsatz erreicht wird.

Nach der Abtrennung des Ionenaustauschers, was üblicherweise durch Sedimentation oder Filtration erfolgen kann oder sich bei Verwendung eines Harzbettes von alleine ergibt, wird das Reaktionsgemisch, soweit erforderlich, von nicht-umgesetztem Acrolein befreit. Die Abtrennung des Acroleins kann in bekannter Weise, insbesondere destillativ, vorzugsweise unter vermindertem Druck und Temperaturen unter 80 °C, durchgeführt werden. Das zurückgewonnene Acrolein kann wieder, gegebenenfalls nach erfolgter Stabilisierung, in den Prozeß zurückgeführt werden. Die erhaltene, nahezu acroleinfreie Hydroxypropionaldehydlösung kann vor der Hydrierung z.B. über einen Dünnschichtverdampfer nochmals aufkonzentriert werden.

Die katalytische Hydrierung des 3-Hydroxypropionaldehyds in flüssiger Phase wird in an sich bekannter Weise und in üblichen Hydrierapparaturen durchgeführt. Der Katalysator kann sowohl in suspendierter Form per se oder trägergebunden zur Anwendung gelangen oder sich in einem Festbettreaktor befinden. Auch homogene Katalysatoren können verwendet werden. Als Suspensionskatalysator sind Raney-Nickel, das mit verschiedenen anderen Metallen dotiert sein kann, sowie feinverteiltes Platin auf einem Trägermaterial wie z.B. Aktivkohle besonders geeignet. Festbettkatalysatoren können die in der US-PS 2,434,110 genannten Stoffe sein. Nickelkatalysatoren haben sich als besonders wirkungsvolle Katalysatoren erwiesen. Zur Erzielung einer hohen Umsatzrate wird die Hydrierung unter Druck und bei erhöhter Temperatur durchgeführt, wobei die wäßrige Lösung einen pH-Wert im Bereich von 3,0 bis 8,5, vorzugsweise um 6, aufweist. Drücke von 20 bis 250 bar, insbesondere 40 bis 140 bar, und Temperaturen von 40 bis 140 °C, insbesondere 60 bis 100 °C, werden bevorzugt.

Die Hydrierung des 3-Hydroxypropionaldehyd kann auch in der Gasphase wie beispielsweise in DE-PS 20 54 601 beschrieben, durchgeführt werden.

Ein weiterer Gegenstand der Erfindung ist ein Ionenaustauscher, welcher dadurch gekennzeichnet ist, daß ein Polyaminharz auf Basis einer vernetzten Polyacrylamid-Matrix mit Acrylsäure, Acrylsäurederivaten oder dem Salz einer ω-Halogenalkansäure umgesetzt wurde.

Vorteile der erfindungsgemäß hergestellten und eingesetzten Hydratisierungskatalysatoren bzw. Ionenaustauscher sind:
1. Sehr niedrige Volumenveränderungen bei der Überführung der erfindungsgemäßen Harze von der Natrium-Form in die H-Form ("Aktivierung") mit verdünnter Säure im Vergleich zu bekannten chelatbildenden Ionenaustauschern vom Iminocarbonsäure-bzw. Aminomethylphosphonsäure-Typ.
2. Die Polyaminstruktur der Katalysatorvorstufe erlaubt eine effiziente Derivatisierung mit hoher Carbonsäuregruppen-Dichte, welche durch die erreichbare Totalkapazität der erhaltenen Harze im Bereich von 0,5 - 8,0 meq.H⁺/ml, vorzugsweise von 1,0 - 5,0⁻meq. H⁺/ml charakterisiert wird. Bei der Verwendung von Monoaminvorstufen (z.B. aminomethylieres Polystyrol/Divinylbenzol-Harz) hingegen ist die erreichbare Totalkapazität aufgrund des monofunktionellen Charakters limitiert.
3. Die erfindungsgemäßen Ionenaustauscherkatalysatoren zeigen in der Hydratisierung von Acrolein zu 3-Hydroxypropionaldehyd gegenüber anderen Ionenaustauschern eine höhere Aktivität und Selektivität (siehe Beispiele).

### Beispiele:

### Beispiel 1

300 g des Polyaminharzes E304/88 (BAYER AG) werden in 1 l 1,4-Dioxan im Rückfluß erhitzt. Das vorgequollene Harz wird abgenutscht und mit destilliertem Wasser gewaschen. Ein 1 l Autoklav wird mit 300 g frisch destillierter, stabilisierter Acrylsäure, 200 g Wasser und dem Harz befüllt, verschlossen und mehrmals mit 15 bar Stickstoff gespült. Anschließend wird das Harz im Autoklaven 3 h bei 70 °C und weitere 2 h bei 130 °C erhitzt. Das Harz wird abgenutscht, mit Wasser gewaschen und zur Aktivierung mit verdünnter Salzsäure (5 Gew.-%) behandelt.
Das erhaltene Harz hat eine Totalkapazität (TK) von 1,8 meq. H⁺/ml und weist keine Volumenveränderung bei der Aktivierung auf.

### Beispiel 2

200 g des Polyaminharzes E304/88 (BAYER AG) werden mehrmals mit destilliertem Wasser gewaschen und abgenutscht. In einem 2 l Dreihalskolben werden 200 g des gewaschenen Harzes, 300 g frisch destillierte Acrylsäure und 200 g Wasser unter schwachem Rühren 6 h bei 65-68 °C erhitzt. Anschließend wird das Harz abgenutscht, mit destilliertem Wasser gewaschen und zur Aktivierung mit verdünnter Salzsäure (5 Gew.-%) behandelt.
Das erhaltene Harz hat eine Totalkapazität (TK) von 2,7 meq H⁺/ml und weist keine Volumenveränderung bei der Aktivierung auf.

### Beispiel 3

Zu einer wäßrigen Lösung aus 35 g (0,33 mol) Natriumcarbonat und 165 g Wasser werden unter Eiskühlung portionsweise 100,5 g (0,64 mol) 3-Brompropionsäure eingetragen. Die erhaltene Mischung wird unter Eiskühlung mit 50 g des gewaschenen Polyaminharzes E304/88 (BAYER AG) versetzt und unter langsamem Erwärmen auf Raumtemperatur 6 h gerührt. Anschließend wird bei 40-60 °C ca. 2 h weitergerührt. Während der Reaktion wird durch kontinuierliche bzw. portionsweise Zugabe gesättigter Natriumcarbonatlösung der pH-Wert der Reaktionsmischung im Bereich von 8-10 konstant gehalten. Nach Beendigung der Reaktion wird das Harz mit voll entsalztem Wasser alkalifrei gewaschen und zur Aktivierung mit verdünnter Salzsäure
(5 Gew.-%) oder Schwefelsäure (2 N) behandelt.
Das erhaltene Harz hat eine Totalkapazität (TK) von
3,21 meq H⁺/ml und zeigt eine Volumenveränderung von ca. -5 % bei der Überführung in die katalytisch aktive H-Form (Aktivierung) auf.

Die Elementaranalyse des Ausgangsharzes und der gebildeten Polyaminpolycarbonsäure-Harze (Beispiele 1-3) ergibt folgende Resultate (Tabelle 1):

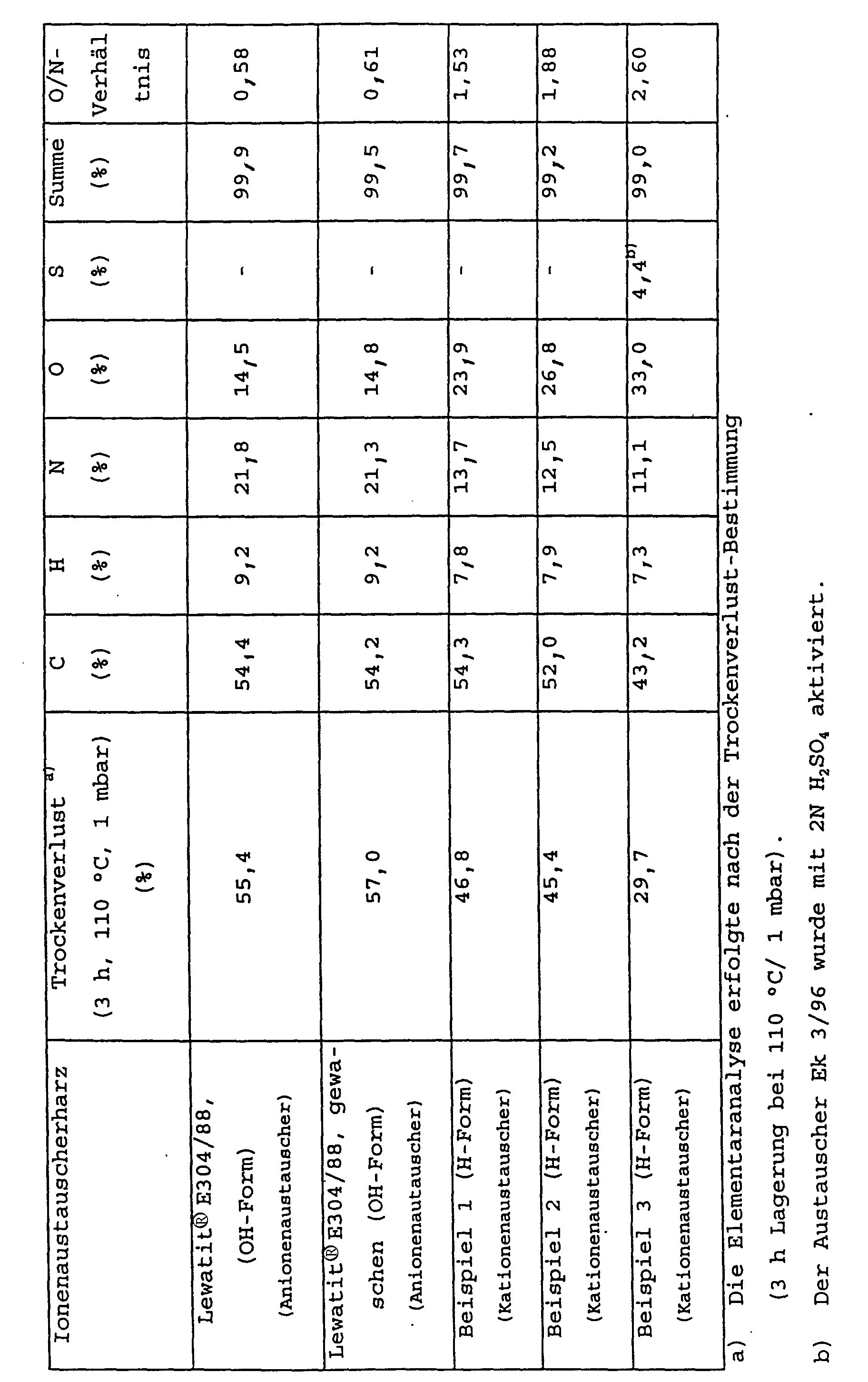

Die erfindungsgemäßen Harze der Beispiele 1-3 weisen einen im Vergleich zum Ausgangsmaterial (E304/88) erhöhten Sauerstoff-Gehalt auf. Die Erhöhung des O/N-Verhältnisses von 0,6 in E304/88 auf 1,5-2,6 (Beispiele 1-3) spiegelt die Derivatisierung mit sauren Funktionen (Carboxylgruppen) wider. Die Analysenergebnisse belegen somit die chemische Modifizierung des Ausgangsharzes.

Die Siebanalyse der Ionenaustauschermuster zeigt, daß sich die Korngröße und die Korngrößenverteilung durch die Derivatisierung nicht verändern: Der Korngrößenbereich (mind. 90%) liegt für E304/88 und die Beispiele 1-3 bei 0,35-1,40 mm. Die effektive Korngröße (Medianwert) beträgt 0,77 ± 0.05 mm.

### Hydratisierung von Acrolein

### Beispiele 4-6 sowie Vergleichsbeispiele (VB 1-4)

Zur Bestimmung der Aktivität der Ionenaustauscher nach dem Stand der Technik (Vergleichsbeispiele VB 1 bis VB 3) sowie der Erfindung (Beispiel 1 bis 3) wird folgender Test durchgeführt:

Ein 20 ml-Septumfläschchen wird mit 10 ml Ionenaustauscher-Katalysator befüllt. Hinzu kommen 14 ml einer wäßrigen Acroleinlösung mit einem Gehalt von 19-20 Gew.-% Acrolein. Die Mischung wird 3 Minuten bei Raumtemperatur durch rotierende Bewegungen geschüttelt und die aktuelle Acrolein-Konzentration einer Probe mittels HPLC bestimmt. Das Septumfläschchen wird gemäß Tab. 1 für die angegebene Zeit im Wasserbad bei der angegebenen Temperatur gerührt und der Reaktionsinhalt anschließend analysiert. Der Acrolein-Umsatz und die Selektivität für 3-Hydroxypropionaldehyd nach 30 und 60 Minuten Reaktion, die Startkonzentration an Acrolein sowie die Reaktionstemperatur sind der Tabelle 2 zu entnehmen.

Ein stark saurer Ionenaustauscher (VB 1) führt zu einem hohen Acrolein-Umsatz, jedoch zu einer unzureichenden Selektivität. Mit dem schwach sauren nicht-chelatbildenden Ionentauscher (VB 2) wird bei niedrigem Umsatz nur eine mittlere Selektivität erhalten. Ein schwach saurer, Ca²⁺-dotierter Ionenaustauscher ist reaktiver, aber ebenfalls nur mittelmäßig selektiv (VB 3). Das nicht derivatisierte Polyaminharz (VB 4), wie in Beispiel 1-3 erwähnt, ist ungeeignet und führt aufgrund des basischen Charakters (OH-Form) zur raschen Polymerisation des Acroleins (sehr niedrige Selektivität). Die erfindungsgemäßen Polyaminpolycarbonsäure-Ionenaustauscher (Beispiel 4-6) hingegen sind sowohl sehr aktiv und als auch sehr selektiv. Auffällig ist der selbst bei großen Umsätzen (> 80 %) geringe Selektivitätsabfall.

### Beispiel 7

In einem Labor-Festbettreaktor mit dem Ionenaustauscherharz aus Beispiel 2 wird die Hydratisierung von Acrolein kontinuierlich über einen längeren Zeitraum durchgeführt. Umsatz und Selektivität werden durch Analyse der Produktlösung bestimmt. Die Apparatur besteht aus einem skalierten, kühlbaren 2-l-Glasgefäß für wäßrige Acrolein-Ausgangslösung, einer HPLC-Pumpe für die Förderung der Reaktionsmischung, einem thermostatisierten, druckbeständigen, präzisionsgeschliffenen Glasrohr (1050 mm x 11,3 mm i.D.),das die Ionenaustauscherfüllung aufnimmt und an beiden Enden mit verstellbaren Verschraubungen verschlossen ist, einem Druckhalteventil, sowie einer auf + 5 °C gekühlten 2-l-Glasvorlage für die Produktlösung und den notwendigen Thermostatisiereinrichtungen.
Das Acrolein-Wasser-Gemisch wird auf + 5 °C gekühlt, vorgelegt und mittels HPLC-Pumpe durch das auf Reaktionstemperatur gehaltene Festbett aus 100 ml Ionentauscherharz bei konstantem Volumenstrom gepumpt. Nach einer Einfahrzeit von 3-5 h zur Einstellung des stationären Zustands wird nach Wechseln der Vorlage unter konstanten Versuchsbedingungen über einen Zeitraum von mehreren Stunden durch das thermostatisierte Glasrohr gepumpt. Diese Messung wird mehrmals wiederholt. Die jeweils erhaltenen Produktlösungen (HPA/Ae) werden mit HPLC analysiert (siehe Tabelle 3).

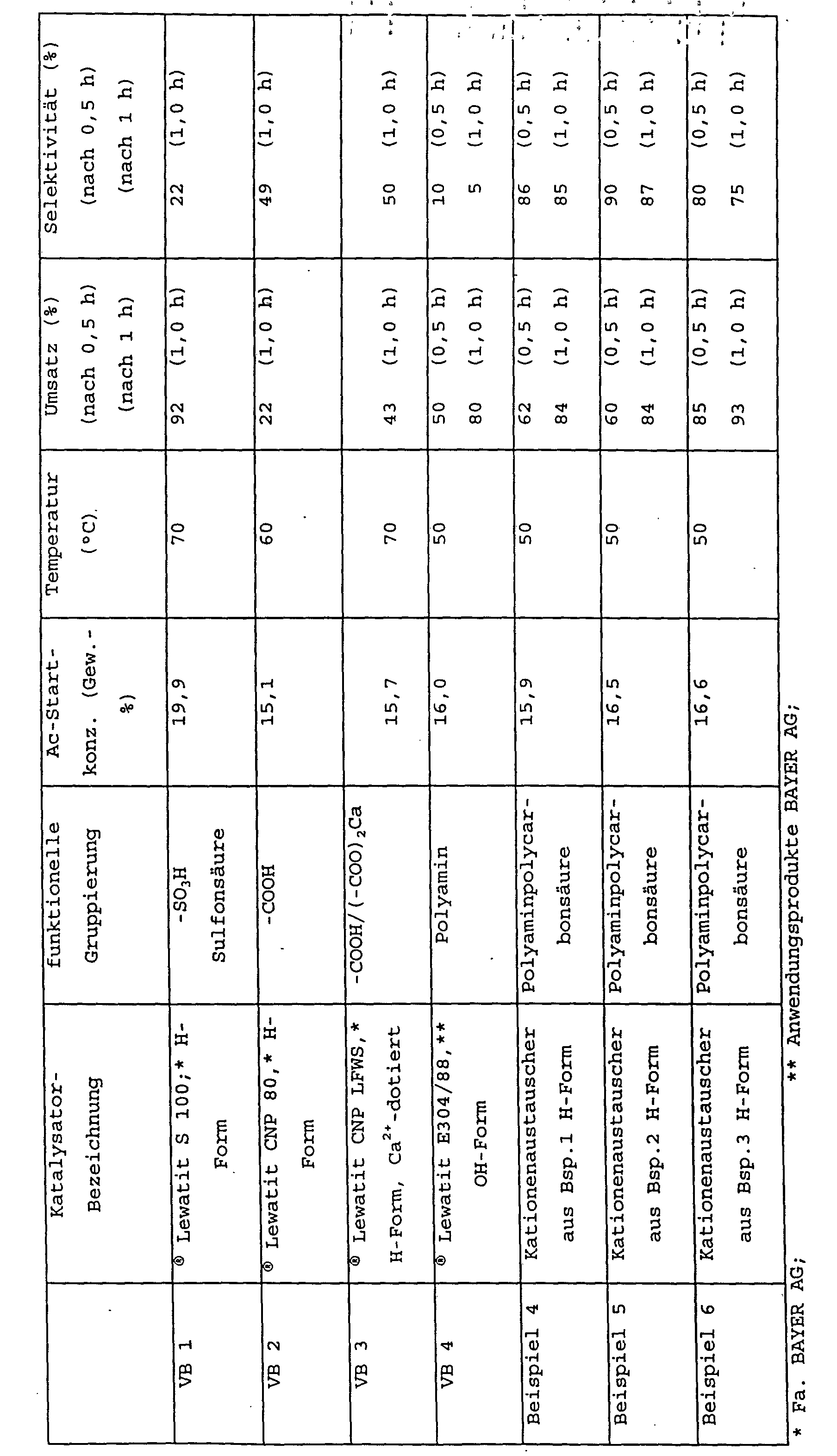

Eingesetzter Ionenaustauscher Polyaminpolycarbonsäure-Harz aus Beispiel 2
Acrolein-Startkonzentration: 18,0 Gew. %,
LHSV (=liquid hourly space velocity): 0,65 h⁻¹

**Tabelle 3**

| Betriebsdauer (Stunden) | Temperatur (°C) | Umsatz (%) | Selektivität (%) |
|---|---|---|---|
| 40 | 45 | 36 | 90 |
| 60 | 50 | 50 | 89 |
| 120 | 60 | 60 | 85 |

### Beispiel 8

Hydrierung einer gemäß Beispiel 7 erhaltenen wäßrigen Lösung von 3-Hydroxypropionaldehyd (HPA) mit Raney-Nickel.

Die Reaktionslösung des Beispiels 7 wird in einer kontinuierlich betriebenen Destillationskolonne mit Dünnschichtverdampfer bei ca. 400 mbar von nicht umgesetztem Acrolein und gleichzeitig einem Teil des Wassers befreit. 500 g der so erhaltenen Lösung werden in einem 1000 ml Autoklaven mit Begasungsrührer bei 135 bar Wasserstoff-Druck, bei einer Temperatur von 75 °C - 140 °C und einer Rührerdrehzahl von 1000 UpM in Gegenwart von 5,8 g Raney-Nickel bei pH 6-7 innerhalb von 60 min hydriert. Der HPA-Umsatz beträgt
99,9 % und die Ausbeute an 1,3-Propandiol 85 %, bezogen auf eingesetztes Acrolein. Die Aufarbeitung der Reaktionslösung erfolgt in an sich bekannter Weise destillativ.

## Patentansprüche

1. Ionenaustauscher, umfassend ein Polyamin-/Polycarbonsäureharz, dadurch gekennzeichnet, daß ein von einer quervernetzten Polyacrylamid-Matrix abgeleitetes Polyaminharz mit Acrylsäure, Acrylsäurederivaten oder Salzen einer ω-Halogencarbonsäure umgesetzt ist, wobei die Polyacrylamid-Matrix durch Reaktion einer Polyacrylatvorstufe mit Tetraethylenpentamin oder Triethylentetramin erhältlich ist.

2. Verfahren zur Herstellung von 1,3-Propandiol durch Hydratisierung von Acrolein in Gegenwart des als Hydratisierungskatalysator verwendeten Ionenaustauschers nach Anspruch 1 unter Bildung von 3-Hydroxypropionaldehyd, wobei Acrolein und Wasser im Gewichtsverhältnis 1:2 bis 1:20 bei 30 bis 120°C und einem Druck im Bereich von 1 bis 20 bar umgesetzt werden, Abtrennung des Ionenaustauschers und, soweit vorhanden, des nicht umgesetzten Acroleins aus dem Reaktionsgemisch und anschließende katalytische Hydrierung des 3-Hydroxypropionaldehyds unter an sich bekannten Bedingungen in flüssiger oder gasförmiger Phase unter Verwendung üblicher Hydrierkatalysatoren.

## Claims

1. Ion exchanger, comprising a polyarnine/polycarboxylic acid resin, characterised in that a polyamine resin derived from a cross-linked polyacrylamide matrix is reacted with acrylic acid, acrylic acid derivatives or salts of a ω-halo acid, whereby the polyacrylamide matrix can be obtained by reacting a polyacrylate precursor with tetraethylenepentamine or triethylenetetramine.

2. Process for the preparation of 1,3-propanediol by hydration of acrolein in the presence of an ion exchanger used as a hydration catalyst according to claim 1, with formation of 3-hydroxypropionaldehyde, whereby acrolein and water are reacted with a weight ratio of 1:2 to 1:20 at 30 to 120°C and a pressure in the range of 1 to 20 bars, separation of the ion exchanger and, in so far as it is present, the non-reacted acrolein from the reaction mixture, and followed by catalytic hydrogenation of the 3-hydroxypropionaldehyde under conditions known per se in liquid or gas phase by using conventional hydrogenation catalysts.

## Revendications

1. Echangeur d'ions comprenant une résine de polyamine/d'acide polycarboxylique, caractérisé en ce qu'une résine de polyamine dérivée d'une matrice de polyacrylamide réticulée est mise en réaction avec de l'acide acrylique, des dérivés de l'acide acrylique ou des sels d'un acide carboxylique oméga-halogéné, la matrice de polyacrylamide pouvant être obtenue par réaction d'un précurseur de polyacrylate avec de la tétraéthylènepentamine ou de la triéthylènetétramine.

2. Procédé de préparation de 1,3-propanediol par hydratation d'acroléine en présence de l'échangeur d'ions selon la revendication 1 utilisé comme catalyseur d'hydratation, avec formation de 3-hydroxypropionaldéhyde, l'acroléine et l'eau étant converties dans un rapport en poids compris entre 1:2 et 1:20 en présence d'une température comprise entre 30 et 120°C et d'une pression allant de 1 à 20 bars, avec séparation de l'échangeur d'ions et de l'acroléine éventuellement non convertie du mélange réactionnel, avant une hydrogénation catalytique ultérieure du 3-hydroxypropionaldéhyde dans des conditions généralement connues, en phase liquide ou gazeuse, avec utilisation de catalyseurs d'hydrogénation usuels.
